# FASCICULE DE BREVET EUROPEEN

(11) **EP 1 140 785 B1**
(45) Date de publication et mention de la délivrance du brevet: **29.12.2004**
(21) Numéro de dépôt: 99958293.5
(22) Date de dépôt: 10.12.1999
(51) Int. Cl.: C07C 209/10, C07C 211/52

(54) **PROCEDE DE PREPARATION DE PARA-TRIFLUOROMETHYLANILINES POLYHALOGENEES**
VERFAHREN ZUR HERSTELLUNG VON POLYHALOGENIERTEN PARA-TRIFLUOROANILINEN
METHOD FOR PREPARING POLYHALOGENATED PARATRIFLUOROMETHYLANILINES

(30) Priorité: 17.12.1998 FR 9816164
(43) Date de publication de la demande: 10.10.2001
(73) Titulaire: BASF Agro B.V. Arnhem (NL)-Wädenswil Branch, 8820 Wädenswil/Au (CH)
(72) Inventeur: ANCEL, Jean-Erick, F-69230 Saint-Genis-Laval (FR); DARNAND, Eliane, F-69005 Lyon (FR)
(74) Mandataire: Köster, Reinhold, Dr.
(86) Numéro de dépôt international: PCT/FR1999/003090
(87) Numéro de publication internationale: WO 2000/035851

(56) Documents cités:
- EP-A- 0 315 869

## Description

La présente invention a pour objet un nouveau procédé de préparation de para-trifluorométhylanilines polyhalogénées, plus particulièrement de dihalogéno-paratrifluorométhylanilines.

Les procédés permettant la préparation d'anilines substituées par un atome d'halogène ont fait l'objet de nombreux travaux ainsi que de nombreux brevets ou publications.

Ainsi, le brevet américain 4 096 185 a pour objet un procédé d'amination de composés aromatiques halogénés, telle la para-trifluorométhylaniline, à partir de para-chloro-trifluorométhylbenzène et avec utilisation d'une combinaison catalytique particulière visant à améliorer le rendement de la réaction.

Le procédé du brevet américain 4 197 259 décrit la préparation d'anilines monohalogénées par la mise en oeuvre de conditions de réaction difficiles liées à l'emploi d'un amidure alcalin comme agent aminant ; l'utilisation d'un tel amidure alcalin exigeant en effet de conduire la réaction en l'absence de toute trace d'eau dans le milieu réactionnel, ces conditions de réaction rendant assez problématique l'industrialisation d'un tel procédé ; de telles conditions de réaction impliquant, en outre, d'importantes difficultés liées à la dissipation de la chaleur de réaction. Le brevet est-allemand 292 238 concerne la préparation d'anilines nitrées et monohalogénées.

La demande de brevet européen 173 202 décrit la préparation de 5-chloro-2-nitroanilines.

Le brevet japonais 5 255 206 et la demande de brevet européen 543 633 ont pour objet la préparation, respectivement, de monofluoro-anilines et de trifluorométhylanilines monofluorées.

Enfin, le brevet européen 315 869 divulgue la préparation de 2,6-difluoro-para-trifluorométhylaniline à partir de 3,4,5-trifluoro-trifluoromethylbenzène à une température de 130°C et avec un rendement de 55%.

Ainsi, et malgré l'importance du nombre des travaux réalisés pour la mise au point de nouvelles voies de synthèse, notamment en vue d'améliorer les procédés connus de préparation d'anilines halogénées, la presque totalité des méthodes connues à ce jour ne concernent que les seules anilines monohalogénées.
En outre, et bien qu'on connaisse par le brevet japonais 7 025 834 un procédé de préparation de nitroanilines polyhalogénées, l'application de ces réactions à des composés substitués par un groupement trifluorométhyle n'est souvent pas transposable du fait, entre autre, de la faible réactivité conférée aux réactifs par ce groupement trifluorométhyle.

L'un des buts de la présente invention est de procurer une méthode de préparation de para-trifluorométhylanilines polyhalogénées.
Un autre but de l'invention est de résoudre les problèmes liés à la préparation d'anilines 2,6-dihalogénées et 4-trifluorométhylées tout en évitant la formation de produits de polyamination ainsi que de produits d'hydrogénation du reste aromatique.
Un but supplémentaire du procédé de l'invention est de favoriser la formation de para-trifluorométhylanilines polyhalogénées plutôt que des isomères metatrifluorométhylés.

Il a maintenant été trouvé que ces objectifs peuvent être atteints en tout ou partie grâce au procédé selon l'invention.
Il s'agit d'un procédé de préparation de dérivés polyhalogénés de la trifluorométhylaniline par réaction trifluorométhylbenzènes polyhalogénés, particulièrement de 3,4,5-trihalogéno-trifluorométhylbenzènes, par exemple de 4-bromo-3,5-dichloro-trifluorométhyl-benzène ou de 3,4,5-trichloro-trifluorométhyl benzène, avec de l'ammoniac.
La réaction du procédé selon l'invention est conduite à une température comprise entre 180 et 270°C et, avantageusement, dans un solvant organique polaire.

Pour la réaction de l'invention on peut, outre l'ammoniac, agir en présence d'un halogénure alcalin.
Ainsi, le procédé selon l'invention permet la préparation des produits de formule (**I**) : 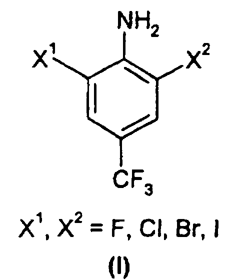 à partir de réactifs de formule **(II)** : 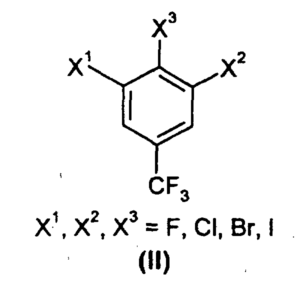 Lorsqu'il est présent, l'halogénure alcalin l'est en quantité catalytique, particulièrement en une quantité comprise entre 1 et 50% de la quantité de réactif de formule **(II)** utilisée, plus particulièrement en une quantité comprise entre 5 et 20%. L'halogénure alcalin est avantageusement un fluorure ou un bromure alcalin, de préférence un halogénure de lithium.

Le procédé selon l'invention est avantageusement mis en oeuvre avec une teneur en eau du milieu réactionnel telle que le rapport H₂O/NH₃ est inférieur à 80/100, de préférence inférieur à 20/100, plus préférentiellement inférieur à 2/100.

Le solvant organique éventuellement mis en oeuvre dans l'invention est, de préférence, un solvant organique polaire, plus préférentiellement un solvant organique polaire non ionique, par exemple une N-alkyl-pyrrolidone dont le radical alkyle possède, de préférence, de 1 à 12 atomes de carbone. L'emploi de la N-méthyl-pyrrolidone est préféré.

La pression qui règne dans le milieu au cours de la réaction selon l'invention est une pression autogène résultant de l'utilisation d'un système fermé, par exemple un autoclave, afin de contrôler au mieux la quantité d'ammoniac présent.

Le produit issu de la réaction selon l'invention est séparé du milieu réactionnel par tout moyen conventionnel, par exemple par distillation, par extraction ou par extraction suivie de distillation ou encore par isolement de sels obtenus par action d'acide chlorhydrique.
En vue d'isoler le dérivé de l'aniline obtenu, le para-trifluorométhylbenzène polyhalogéné n'ayant pas réagi est séparé du milieu réactionnel. Dans le cas où le dérivé du benzène utilisé n'est présent qu'en faible quantité, cette étape peut être omise.
En menant la réaction du procédé selon l'invention dans un réacteur équipé d'un appareillage de distillation, le dérivé de l'aniline visé et le dérivé du benzène n'ayant pas réagi sont directement séparés du milieu réactionnel par distillation.
Pour isoler le produit de réaction visé par extraction, on ajoute au milieu réactionnel de l'eau et un solvant organique à bas point d'ébullition, par exemple de l'éther, du dichlorométhane ou de l'héxane. Après séparation de la phase aqueuse, on distille la phase organique afin de séparer le dérivé de l'aniline visé du dérivé du benzène n'ayant pas réagi. Le dérivé du benzène ainsi séparé est alors recyclé comme réactif pour le procédé de l'invention.
Un autre mode de séparation du produit de réaction de l'invention est sa salification par action d'acide chlorhydrique. Pour cela, on dilue le mélange réactionnel dans un solvant dans lequel le dérivé salifié de l'aniline visé est peu soluble, on fait ensuite agir un flux d'acide chlorhydrique dans le milieu réactionnel afin de faire précipiter le dérivé de l'aniline visé ainsi salifié. On filtre ensuite le précipité obtenu.

Le procédé de l'invention permet la préparation des dérivés de l'aniline envisagés avec un taux de conversion des réactifs particulièrement avantageux. Les performances du procédé de l'invention varie cependant selon les conditions réactionnelles particulières choisies ; l'homme de l'art peut facilement trouver des conditions de réaction optimales en suivant les indications de la présente description.
Un autre avantage du procédé de l'invention est de permettre la transformation des trifluorométhylbenzènes polyhalogénés en dérivés de l'aniline avec un rendement élevé.
Ce procédé est également particulièrement avantageux en ce qu'il donne accès à une haute sélectivité favorisant la formation des para-trifluorométhylanilines polyhalogénées plutôt que des meta-trifluorométhylanilines polyhalogénées.

Un avantage supplémentaire du procédé de l'invention est qu'il permet des temps de réaction pouvant être courts ; par exemple des temps de réaction inférieurs à 10 heures, voire, des temps de réaction inférieurs à 5 heures peuvent être mis en oeuvre.

Parmi les trifluorométhylanilines polyhalogénées dont le procédé selon l'invention permet la préparation, la 2,6-dichloro-4-trifluorométhylaniline est particulièrement intéressante comme intermédiaire de réaction pour la préparation de composés utilisés comme insecticides.

Les différents exemples qui suivent permettront de mieux illustrer le procédé de l'invention ainsi que les avantages qui y sont attachés ; toutefois, ces exemples ne limitent en rien l'étendue de l'invention.

### Exemple n°1 :

Dans un autoclave, on mélange du 3,4,5-trichloro-trifluorométhylbenzène (0,681g, 2,73mmol), du fluorure de lithium (7,1mg, 0,273mmol) et de la N-méthyl-pyrrolidone (1,8ml). On refroidit à -95°C puis on ajoute de l'ammoniac (1,3g, 76.4mmol). On chauffe alors à 250°C et sous agitation pendant 4h, on laisse ensuite revenir à température ambiante.
On extrait le produit de réaction par un lavage à l'eau et au dichlorométhane puis on évapore après avoir séché la phase organique.
On obtient 0,453g (1,97mmol) de 2,6-dichloro-4-trifluorométhylaniline avec un taux de conversion du 3,4,5-trichloro-trifluorométhylbenzène de 97%, un ratio égal à 83/17 pour la sélectivité en 2,6-dichloro-para-trifluorométhyl-aniline par rapport à la 2,6-dichloro-meta-trifluorométhyl-aniline et un rendement de 87% en 2,6-dichloro-para-trifluorométhylaniline.

### Exemple n°2 :

Dans un autoclave, on mélange du 4-bromo-3,5-dichloro-trifluorométhylbenzène (0,802g, 2,73mmol) et de la N-méthyl-pyrrolidone (1,8ml). On refroidit à -95°C puis on ajoute de l'ammoniac (1,3g, 76.4mmol). On chauffe alors à 200°C et sous agitation pendant 4h, on laisse ensuite revenir à température ambiante.
On extrait le produit de réaction par un lavage à l'eau et au dichlorométhane puis on évapore après avoir sécher la phase organique.
On obtient 0,428g (1.86mmol) de 2,6-dichlro-4-trifluorométhylaniline avec un taux de conversion du 4-bromo-3,5-dichloro-trifluorométhylbenzène de 91%, une sélectivité totale en 2,6-dichloro-para-trifluorométhylaniline et un rendement de 68% en 2,6-dichloro-para-trifluorométhylaniline.

## Revendications

1. Procédé de préparation de produits de formule **(I)** : 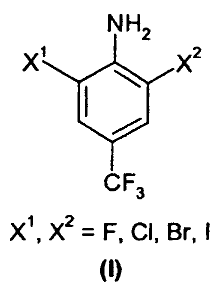 à partir de réactifs de formule **(II)** : 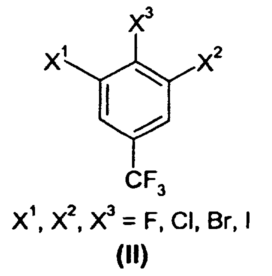 en présence d'ammoniac et à une température comprise entre 180 et 270°C.

2. Procédé selon la revendication 1 de préparation de produits de formule **(I)** : 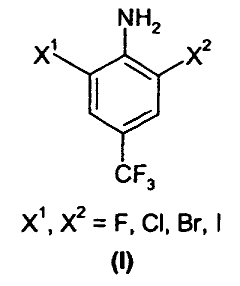 à partir de réactifs de formule **(II)** : 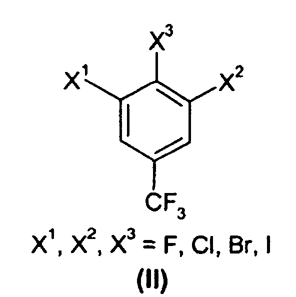 en présence d'ammoniac, d'un halogénure alcalin et à une température comprise entre 180 et 270°C.

3. Procédé selon la revendication 2 dans lequel l'halogénure alcalin est présent en quantité catalytique.

4. Procédé selon la revendication 2 dans lequel l'halogénure alcalin est présent en une quantité comprise entre 1 et 50%, de préférence entre 5 et 20%, de la quantité molaire de réactif de formule **(II)**.

5. Procédé selon l'une ou l'autre des revendications 1 à 4 pour lequel la teneur en eau du milieu réactionnel est telle que le rapport H₂O/NH₃ est inférieur à 80/100, de préférence inférieur à 20/100, plus préférentiellement inférieur à 2/100.

6. Procédé selon l'une ou l'autre des revendications 1 à 5 pour lequel est utilisé un solvant organique polaire, de préférence un solvant organique polaire non ionique.

7. Procédé selon l'une ou l'autre des revendications 1 à 6 pour lequel le solvant employé est une N-alkyl-pyrrolidone dont le radical alkyle possède de préférence de 1 à 12 atomes de carbone, plus préférentiellement la N-méthyl-pyrrolidone.

## Patentansprüche

1. Verfahren zur Zubereitung von Produkten nach der Formel (I): 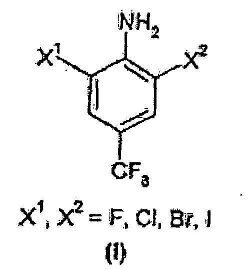 von Reaktionspartnern der Formel (II) aus: 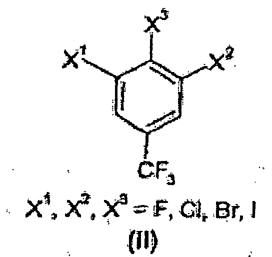 in Anwesenheit von Ammoniak und bei einer Temperatur zwischen 180 und 270°C.

2. Verfahren nach Anspruch 1 zur Zubereitung von Produkten der Formel (I): 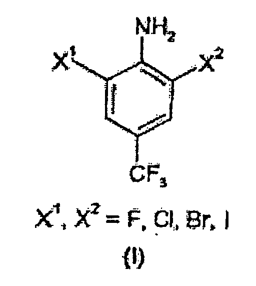 von Reaktionspartnern der Formel (II) aus: 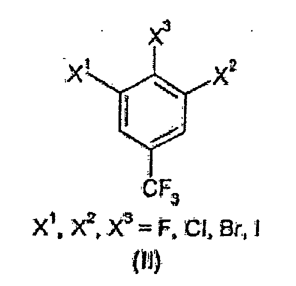 in Anwesenheit von Ammoniak, einem alkalischen Halogenid und bei einer Temperatur zwischen 180 und 270°C.

3. Verfahren nach Anspruch 2, wobei das alkalische Halogenid in katalytischer Menge vorliegt.

4. Verfahren nach Anspruch 2, wobei das alkalische Halogenid in einer Menge zwischen 1 und 50 %, vorzugsweise zwischen 5 und 20 % der molaren Menge des Reaktionspartners der Formel (II) vorliegt.

5. Verfahren nach irgendeinem der Ansprüche 1 bis 4, wobei der Gehalt an Wasser des Reaktionsmilieus derart ist, dass das Verhältnis H₂O/NH₃ unter 80/100, vorzugsweise unter 20/100, noch bevorzugter unter 2/100 beträgt.

6. Verfahren nach irgendeinem der Ansprüche 1 bis 5, wobei ein polares organisches Lösungsmittel, vorzugsweise ein nichtionisches, polares organisches Lösungsmittel verwendet wird.

7. Verfahren nach irgendeinem der Ansprüche 1 bis 6, wobei das verwendete Lösungsmittel ein N-Alkylpyrrolidon, dessen Alkylradikal vorzugsweise 1 Kohlenstoffatom bis 12 Kohlenstoffatome besitzt, vorzugsweise das N-Methylpyrrolidon ist.

## Claims

1. A process for the preparation of products of formula (I): 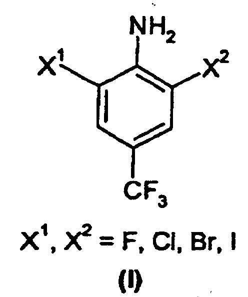 from reactants of formula (II): 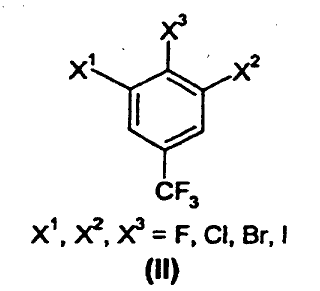 in the presence of ammonia and at a temperature of between 180 and 270°C.

2. The process as claimed in claim 1 for the preparation of products of formula (I): 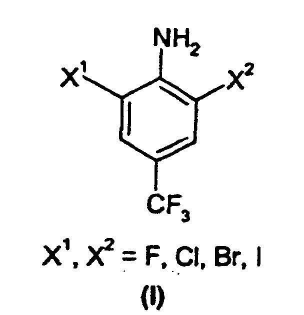 from reactants of formula (II): 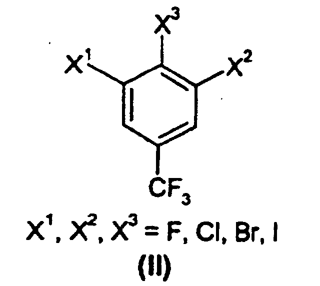 in the presence of ammonia and of an alkali metal halide and at a temperature of between 180 and 270°C.

3. The process, as claimed in claim 2, in which the alkali metal halide is present in a catalytic amount.

4. The process as claimed in claim 2, in which the alkali metal halide is present in an amount of between 1 and 50%, preferably between 5 and 20%, of the molar amount of reactant of formula (II).

5. The process as claimed in any of claims 1 to 4, for which the content of water in the reaction medium is such that the H₂O/NH₃ ratio is less than 80/100, preferably less than 20/100, more preferably less than 2/100.

6. The process as claimed in any of claims 1 to 5, for which a polar organic solvent, preferably a nonionic polar organic solvent, is used.

7. The process as claimed in any of claims 1 to 6, for which the solvent employed is an N-alkylpyrrolidone, the alkyl radical of which preferably has from 1 to 12 carbon atoms, more preferably N-methylpyrrolidone.
